# EUROPEAN PATENT SPECIFICATION

(11) **EP 2 280 750 B1**
(45) Date of publication and mention of the grant of the patent: **20.11.2013**
(21) Application number: 09734495.6
(22) Date of filing: 13.01.2009
(51) Int. Cl.: A61M 5/168

(54) **FLOW SENSOR CONTROLLED INFUSION DEVICE**
MITTELS FLUSSSENSOR GESTEUERTE INFUSIONSVORRICHTUNG
DISPOSITIF DE PERFUSION COMMANDÉ PAR UN DÉTECTEUR D'ÉCOULEMENT

(30) Priority: 24.04.2008 US 108605
(43) Date of publication of application: 09.02.2011
(73) Proprietor: Medtronic, Inc, Minneapolis, MN 55432 (US)
(72) Inventor: SEELEY, Dale F., Spring Park MN 55384 (US); DENISON, Timothy J., Minneapolis Minnesota 55410 (US)
(74) Representative: Hughes, Andrea Michelle
(86) International application number: PCT/US2009/030837
(87) International publication number: WO 2009/131719

(56) References cited:
- WO-A-97/40872
- GB-A- 2 127 179
- US-A- 4 443 218

## Description

### FIELD

This disclosure relates to implantable medical devices, particularly implantable infusion devices employing flow sensors to regulate flow rate.

### BACKGROUND

A wide variety of implantable infusion devices are available for delivering fluid to target locations of a patient into which the device is implanted. Available and proposed devices can differ in their ability to control the flow rate of fluid delivered from the device to the patient. For example, Medtronic Inc.'s (Minneapolis, MN) SYNCHROMED series of implantable infusion devices are programmable devices where the flow rate may be varied according to instructions provided by, e.g., a physician programmer device. Medtronic Inc.'s SYNCHROMED implantable infusion devices employ peristaltic pumps that expel discrete amounts or spurts of fluid and can provide a wide range of fluid flow rates. Regardless of the pumping mechanism employed, fully programmable infusion devices are typically active devices requiring constant or near constant energy. The energy is typically supplied by a battery source, which increases the size of and cost to manufacture of the device. While fully programmable infusion devices allow for a wide variety of flow rates, they do at the expense of energy, size and design simplicity.

Other devices, such as Medtronic, Inc.'s ISOMED implantable infusion device, are configured to deliver a relatively constant rate of fluid to the patient. Such constant rate devices are typically passive and are relatively simple in their components and construction. For example, the ISOMED device employs a fluid propellant source to force fluid out of a bellows reservoir and employs a capillary flow restrictor downstream of the positive pressure reservoir to control flow rate. The flow rate is dependent upon the fluidic resistance of the flow restrictor, temperature and viscosity of the fluid and the pressure differential across the restrictor, with pressure on one side being determined essentially by the pressure in the reservoir and pressure on the other side being determined essentially by body pressure, which closely follows atmospheric pressure. If any of fluidic resistance, temperature, viscosity or atmospheric pressure changes, the fluid flow rate can change. For example, changes in temperature or pressure associated with normal use of such devices can change flow rate 10-20%, which is unacceptable for a variety of therapies. Pressure regulator devices have been proposed to counteract the effect of ambient pressure change. However, the ability of such pressure regulators to counteract pressure change in a reliably consistent manner over time is in doubt. In addition, pressure regulators can require very precise/complex parts and fabrication to perform adequately.

Other infusion devices have been proposed that employ valves and a series of flow restrictors to convert a constant flow rate device into a selectable flow rate device. Such devices attempt to marry the simplicity of a constant rate device with the features of a fully programmable device. Some of the proposed selectable rate devices employ pressure sensors to determine the appropriate valves to open and close to direct fluid through a flow path with one or more flow restrictors to achieve a desired flow rate. However, such devices are still susceptible to changes in fluidic resistance, temperature, viscosity and pressure described above regarding constant rate infusion devices. That is, flow rate across any given flow restrictor may vary with, for example, a change in atmospheric pressure. Changes in pressure can result in feedback-controlled changes in flow path across a different flow restrictor, causing the device to make many active adjustments. Further, to account for differences in flow rate due to viscosity, temperature, and the like would require further components and design considerations in such devices, making their complexity more like programmable infusion devices.

In summary, programmable pumps often perform at a high level but typically require more energy, components, size and cost. Constant rate pumps are simple but do not work well in changing environments without adding complexity such as regulators. Selectable rate pumps loose some of the simplicity of the constant rate pumps and then become less differentiated from programmable pumps.

GB 2127179 teaches an implantable infusion device with a flow regulator.

### BRIEF SUMMARY

The present disclosure describes, among other things, implantable infusion devices having a flow sensor feedback controlled fluid flow rate. The devices employ an actuator mechanism for controlling the relative pressure across a diaphragm of a pressure regulator or controlling a variable resistance valve to control flow rate. The devices may be used to provide a constant delivery rate or a variable delivery rate. In many embodiments, the complexity and manufacturing challenge of some of the components may be reduced due to the flow sensor feedback control.

In various embodiments, an implantable infusion device is described. The infusion device includes an outlet through which a fluid is deliverable and a reservoir for containing the fluid. A flow path is in fluid communication with the reservoir and the outlet. The flow path includes a pressure regulator and a flow restrictor. The pressure regulator has a housing defining a major chamber and a diaphragm disposed in the housing such that the diaphragm sealingly divides the major chamber into first and second minor chambers. The flow restrictor is in fluid communication with the first and second minor chambers of the pressure regulator and is disposed downstream of the first minor chamber and upstream of the second minor chamber. The device further includes (i) a flow sensor configured to detect information regarding flow rate of the fluid downstream of the flow restrictor, and (ii) an actuator assembly configured to vary pressure in the first minor chamber of the pressure regulator relative to pressure in the second minor chamber. The device also includes a processor operably coupled to the flow sensor and the actuator assembly. The processor is configured to provide instructions to the actuator assembly for adjusting the pressure in the first minor chamber relative to pressure in the second minor chamber based on the information from the sensor to regulate flow rate of the fluid. In some embodiments described herein, the pressure regulator is replaced by a variable valve configured to restrict fluid flow through the valve to varying degrees. The actuator assembly may be employed to adjust the degree to which the variable valve is opened.

In various embodiments, a method for controlling a flow rate of a fluid through a flow path of an implantable infusion device is described. The flow path includes a flow restrictor and a flow regulator. The flow regulator includes a major chamber sealingly divided into first and second minor chambers by a diaphragm. The flow restrictor is disposed in the flow path between the first and second minor chambers. The method includes sensing flow rate information downstream of the flow restrictor and determining whether the flow rate is at a target rate based on the sensed information. The method further includes adjusting the relative pressure between the first and second minor chambers of the pressure regulator if the flow rate is not at the target rate. If a variable valve is employed in place of a pressure regulator, the degree to which the valve is open may be adjusted.

Various embodiments of the present invention provide several advantages over known methods and apparatuses. For example, some of the embodiments described herein, provide for devices having fewer parts requiring tight tolerances than devices described previously. Such devices may, in some circumstances, be easier to manufacture than previously described implantable infusion devices. By providing a pressure adjustment actuator assembly coupled to a diaphragm of a flow regulator, a constant rate of delivery may be maintained over time, as the biasing force applied the diaphragm can be adjusted. Similarly, variable flow rate can be achieved with a high degree of accuracy by altering biasing force applied to the diaphragm. By employing a variable vale operably coupled to an adjustment actuator similar results may be obtained. These and other advantages will be evident to one of skill in the art upon reading the disclosure herein.

### BRIEF DESCRIPTION OF THE DRAWINGS

**FIG. 1** is a schematic diagram showing an embodiment of an infusion device and operably coupled catheter implanted in a patient.
**FIGs. 2-6** are schematic block diagrams of some components of implantable infusion devices showing representative flow paths and control components.
**FIGs. 7A-B** are schematic hybrids of block diagrams and cross-sectional views of an example of an actuator assembly and pressure regulator.
**FIG. 8** is a flow diagram of a method for controlling a fluid flow rate of an implantable infusion device.
**FIG. 9** is a flow diagram of a method for controlling a fluid flow rate of an implantable infusion device.

The drawings are not necessarily to scale. Like numbers used in the figures refer to like components, steps and the like. However, it will be understood that the use of a number to refer to a component in a given figure is not intended to limit the component in another figure labeled with the same number. In addition, the use of different numbers to refer to components is not intended to indicate that the different numbered components cannot be the same or similar.

### DETAILED DESCRIPTION

In the following detailed description, reference is made to the accompanying drawings that form a part hereof, and in which are shown by way of illustration several specific embodiments of devices, systems and methods. It is to be understood that other embodiments are contemplated and may be made without departing from the scope of the present disclosure. The following detailed description, therefore, is not to be taken in a limiting sense.

All scientific and technical terms used herein have meanings commonly used in the art unless otherwise specified. The definitions provided herein are to facilitate understanding of certain terms used frequently herein and are not meant to limit the scope of the present disclosure.

As used in this specification and the appended claims, the singular forms "a", "an", and "the" encompass embodiments having plural referents, unless the content clearly dictates otherwise. As used in this specification and the appended claims, the term "or" is generally employed in its sense including "and/or" unless the content clearly dictates otherwise.

The present disclosure describes, among other things, flow paths and control mechanisms for controllable rate infusion devices. The components described herein may be employed in a wide number of implantable infusion devices for delivering fluid to one or more target location of a patient in which the infusion device is implanted.

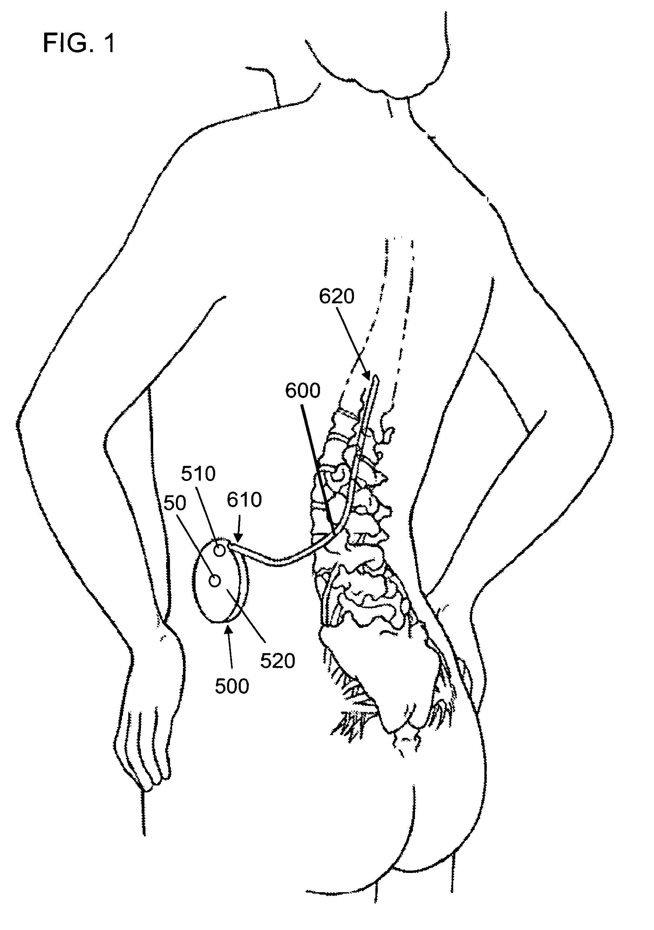

In various embodiments the implantable infusion device has a hermetically sealed housing in which some or all of the components are stored. For example and referring to **FIG. 1**, an implantable infusion device **500** and associated catheter **600** is shown implanted in a patient. In the depicted embodiment, the device **500** is implanted in a subcutaneous pocket in an abdominal region of the patient. However, it will be understood that the device may be implanted in any medically acceptable location of the patient. The device **500** includes a hermetically sealed housing **520** and a refill port **50** accessible from outside the housing **520**. The refill port **50** provides access to the reservoir (not shown in **FIG. 1**). The reservoir may be refilled by percutaneously inserting a needle (not shown) into the patient such that needle enters refill port **50,** and fluid may be delivered into reservoir from needle via refill port **50**. The depicted device **500** also includes a catheter access port **510** in fluid communication with the catheter **600**. Fluid may be injected into or withdrawn from the patient through catheter **600** via catheter access port **510** by percutaneously inserting a needle into access port **510**. Catheter **600** is typically a flexible tube with a lumen running from the proximal end of catheter **600** to one or more delivery regions that are typically located at the distal portion of catheter **620**. Proximal portion **610** of catheter **600** is connected to infusion device **500**. Distal portion **620** of catheter **600** is positioned at a target location in the patient to deliver fluid from infusion device **500** to patient through a delivery region of catheter **600**. While the system depicted in **FIG. 1** is implanted to deliver fluid from the device **500** to the patient intrathecally via the catheter **600**, it will be understood that the fluid can be delivered to any desired location.

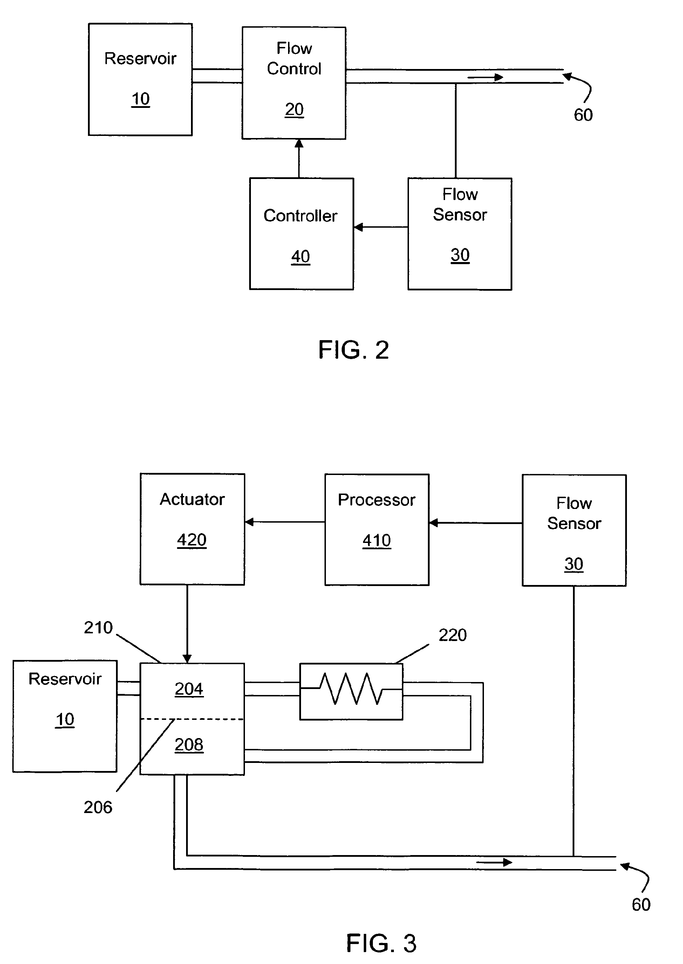

Referring now to **FIG. 2**, an overview of selected components of a controllable rate infusion device are shown in block diagram form. The device includes a reservoir **10** for storing fluid, which typically is a liquid composition including a therapeutic agent, and an outlet **60** through which the fluid can be delivered. The device includes a flow path extending from the reservoir **10** to the outlet **60**. For purposes of the present disclosure, the reservoir **10** is discussed as being "upstream" of the outlet **60** in the flow path. The flow path includes a flow control mechanism **20** downstream of the reservoir **10** to control the flow rate of fluid delivered through the outlet **60**. The device further includes a flow sensor **30** capable of detecting information regarding the flow rate of fluid that will exit opening **60**. The flow sensor **30** may detect any information from which flow rate may be derived or estimated. For example, the flow sensor **30** may detect pressure upstream and downstream of a flow restrictor (not shown), may detect temperature upstream and downstream of a heating element (not shown), such as with a mass flow sensor, or the like. The device further includes a controller **40**, such as processor or series of processors, which is capable of causing the flow control mechanism **20** to be adjusted based on information obtained from the sensor **30** to modify flow rate.

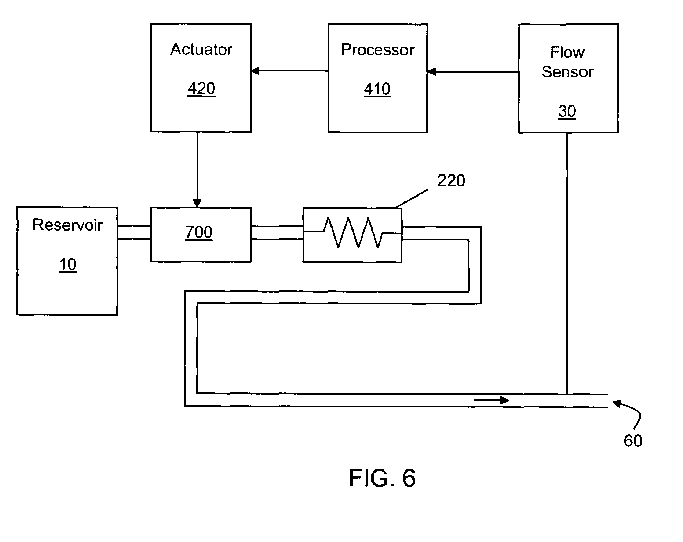

It will be readily apparent to those of ordinary skill in the art that a multitude of configurations of infusion devices including a reservoir, a flow control mechanism, a fluid flow path, a controller and a flow sensor may be employed to obtain a suitable controllable rate implantable infusion device. Examples of some configurations of such devices, or at least selected components of such devices, are shown in **FIGs. 3-6**. **FIGs. 3-6** include additional details, relative to **FIG. 2**, of various embodiments of components of controllable rate infusion devices. In **FIGs. 3-5**, the flow path includes a pressure regulator **210** and flow restrictor **220**. The pressure regulator **210** has a housing defining a major chamber and a diaphragm **206** disposed in the major chamber. The diaphragm **206** sealingly divides the major chamber into first **204** and second **208** minor chambers. The flow restrictor **220** is in fluid communication with the first **204** and second **208** minor chambers and is located in the flow path downstream of the first chamber **204** and upstream of the second chamber **208**. The flow control mechanism includes an actuator assembly **420** configured to cause the diaphragm **206** to flex to control the relative pressure between the first chamber **204** and the second chamber **208** to control the rate of fluid flow through the flow path. Details regarding an embodiment of the interaction of the actuator assembly **420** and the pressure regulator **210** will be described below with regard to **FIGs. 6A-B**. The actuator assembly **420** in the embodiments depicted in **FIGs. 3-5** receives instructions from processor **410** to change the position or bias on the diaphragm **206** to a greater or lesser degree. The processor **410** receives information from the flow sensor and instructs the actuator assembly **420** based on the information from the sensor.

It will be understood that regulator diaphragms without actuator adjustment are configured to change position based on pressure changes. However, with the actuator adjustment described herein, manufacturing of the pressure regulator and diaphragm may be less precise, allowing for the pressure regulator to provide gross adjustment with more precise adjustment provided by the actuator assembly based on flow sensor feedback. The configurations described herein also allow for improved performance over time, as the actuator assembly may compensate for degradation in material or system performance of the pressure regulator over time.

Any suitable flow restrictor **220** may be used in accordance with the embodiments depicted in **FIGs. 3-6**. For example, a flow restrictor may be a fluid conduit of restricted diametric dimension or a media to resist fluid flow. In some embodiments, the flow restrictor **220** is a capillary tube. In some embodiments, the flow restrictor **220** includes a fluidic path micro-machined in glass or silicon.

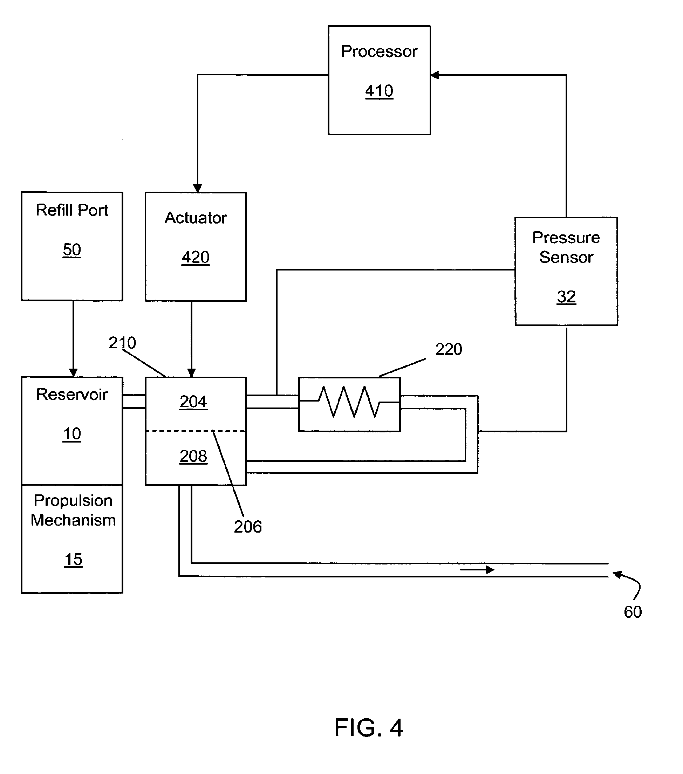

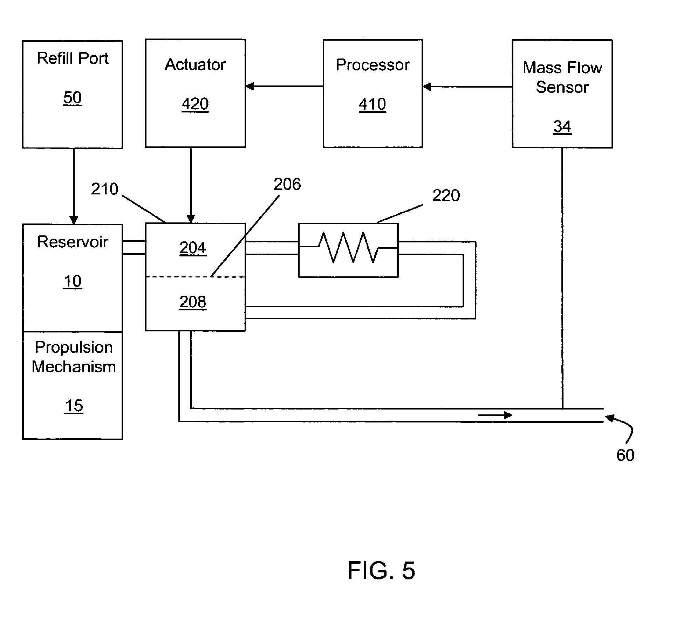

As depicted in **FIGs. 4** and **5**, a propulsion mechanism **15** may be operably coupled to the reservoir **10** to drive fluid out of the reservoir **10**. Any suitable propulsion mechanism **15** may be employed. By way of example, the reservoir **10** may be a bellows reservoir and the propulsion mechanism **15** may contain a propellant chamber that contains a fluid whose vapor pressure is such that, under conditions of normal body temperature, pressure is exerted on the bellows to force liquid in the reservoir **10** to enter the pressure regulator **210**. Examples of such propulsion mechanisms are found in Medtronic Inc.'s SYNCHROMED and ISOMED implantable infusion devices. A mechanical spring may be readily substituted for the liquid propellant. Alternatively, reservoir **10** may be formed, at least in part, of an elastomeric or resilient material biased in an empty configuration that expands when filled and forces fluid to exit reservoir **10** and enter regulator **210**. Thus, the propulsion mechanism **15** and reservoir **10** may, in some embodiments, be the same component.

As shown in **FIGs. 4** and **5**, a refill port **50** may be included in an infusion device. The refill port **50** is in fluid communication with the reservoir **10** and provides access to the reservoir **10** to allow liquid to be delivered to, or withdrawn from, the reservoir **10**. The refill port **50** may include a one-way valve mechanism (not shown) that allows fluid to be delivered to the reservoir **10** but prevents fluid from escaping the reservoir **10** via the access port **50**. The refill port **50** may include check valve (not shown) or other mechanism to prevent overfilling of reservoir. One or more sensors (not shown) may be employed to detect needle entry into port **50**, provide feedback regarding fill status of the reservoir **10**, or the like.

As depicted in **FIG. 4**, flow sensor may include one or more pressure sensors **32**. In the depicted embodiment, pressure sensor **32** senses pressure upstream and downstream of flow restrictor **220**. Information regarding flow rate can be readily obtained by comparing pressure upstream and downstream of flow restrictor **220**. Given a known viscosity of fluid flowing through the restrictor **220**, the flow rate can readily be calculated by known algorithms based on the pressure differential across the restrictor **220**. However, viscosity of fluid to be delivered to a patient from the reservoir **10** may depend on the composition of the fluid, internal body temperature of the patient into which the infusion device is implanted, or the like. Information regarding viscosity of the fluid composition may be provided to the infusion device by, e.g., an external device (not shown) in telemetric communication with the infusion device, for example when the reservoir **10** is refilled. The information may be stored in memory (not shown) and retrieved by processor **410** for use in determining whether or how much adjustment is appropriate and instructing actuator element **420** accordingly. A temperature sensor (not shown) may also be employed to provide information to processor **410**, e.g. through memory or in real time, to account for a change in viscosity that may have occurred. While one pressure sensor **32** is depicted in **FIG. 3**, it will be understood that two pressure sensors may be employed, one upstream and one downstream of flow restrictor **220**, both of which are operably coupled to processor **410**. In some embodiments, pressure sensor **32** is a pressure transducer operably coupled to the flow path upstream and downstream of the restrictor **410**. Of course, any suitable pressure sensor device may be employed.

As depicted in **FIG. 5**, flow sensor includes a mass flow sensor **34**. Mass flow sensor **34** may be any suitable mass flow sensor. For example, the mass flow sensor **34** may include a heating or resistive element and two temperature sensors for measuring temperature of fluid upstream and downstream of the element. The sensor **34** (e.g. with on-board processor) or processor **410** may correlate the temperature difference to liquid mass flow. Such mass flow sensors are described in, for example, US Patent No. 6,813,944 to Mayer et al., issued on Nov. 9, 2004, assigned to Sensirion AG, and entitled "FLOW SENSOR", which describes mass flow sensors that can detect liquid flow rates through a variety of materials. Such sensors may be desirable to limit the number of components that come into contact with the liquid composition to be delivered to a patient. By limiting the number of components coming into contact with the liquid composition, concerns regarding the choice of material are reduced. For example, considerations such as whether the component may be affected by the liquid composition, whether the liquid composition may be affected by the component, and whether any safety concerns are presented in delivering a liquid that has contacted the component to a patient are reduced or eliminated if the component is not in contact with the liquid composition.

In some embodiments a pressure sensor device **32**, e.g. as depicted in **FIG. 4**, is employed to provide information regarding fluid flow rate. In some embodiments a mass flow sensor device **34**, e.g. as depicted in **FIG. 5**, is employed to provide information regarding fluid flow rate. As mass flow sensors provide a more direct measurement of flow rate that is not dependent on a correlation of pressure, temperature or fluidic resistance, infusion devices employing mass flow sensors may prove to be a bit more accurate.

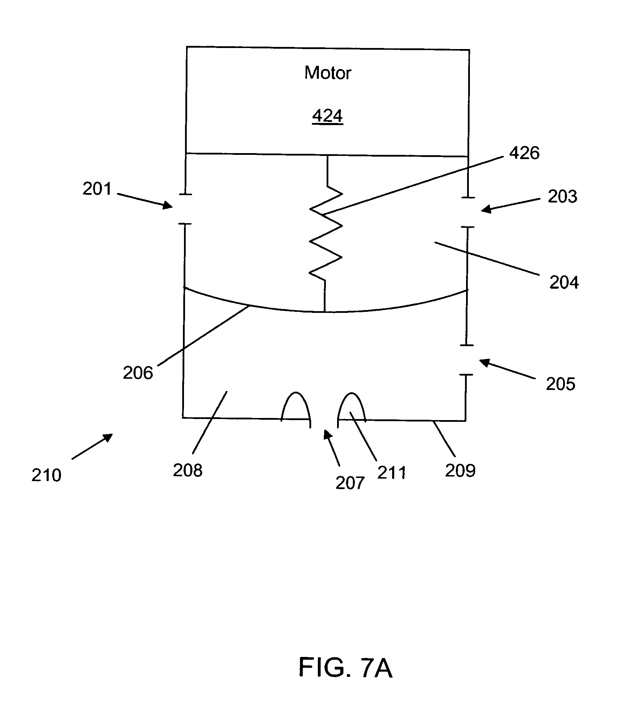

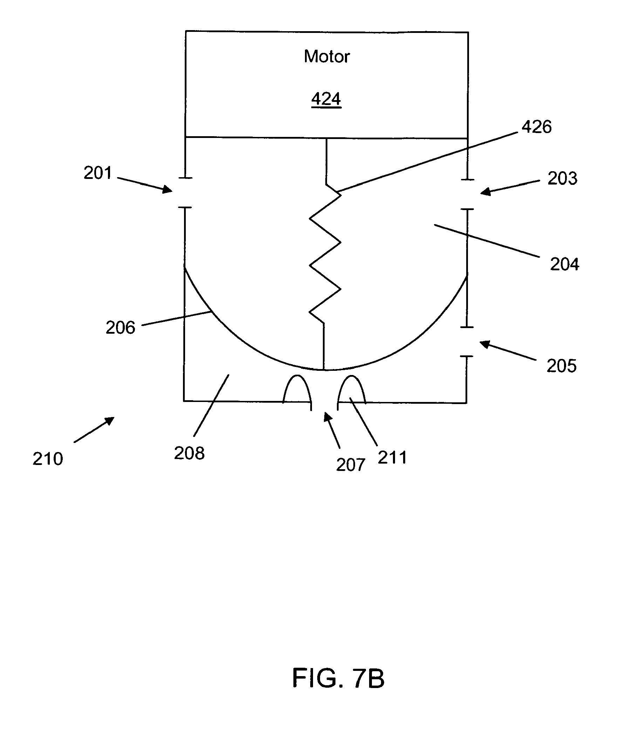

In various embodiments, a pressure regulator **210** as described above may be replaced with a variable valve. For example, and referring to **FIG. 6**, a variable valve **700** may be located upstream of a flow restrictor **220**. Flow sensor **30** may detect information relating to flow rate and provide such information to processor **410**, which in turn may instruct actuator **420** to adjust the amount that the valve is opened or closed. Any suitable actuatable valve assembly may be employed. For example, the valve may resemble a needle valve in a carburetor jet where a tapered needle is advanced into or withdrawn from a tapered opening to control flow. A ball valve or any other suitable valve may also be employed. The actuator mechanism **420** can be instructed to adjust the size of the opening of the variable valve **700** to change the rate at which fluid can flow through the valve **700**. Otherwise, the components of the flow path, sensor **30**, reservoir **10**, processor **410** and actuator **420** depicted in **FIG. 6** may be similar to those discussed above (e.g. with regard to **FIGs. 2-5**) or below (e.g. with regard to **FIGs. 7A**-**B**).

It will be understood that additional components that are not depicted in **FIGS. 1-6** may be incorporated into an infusion device. For example, the device may include a power supply for powering the sensor **30**, processor **410**, actuator assembly **420** or the like. The device may include memory for storing information accessible by processor **410**, storing information obtained from sensor **30**, or the like. The device may include a telemetry module for communicating with a remote device, such as a programmer device, for example to obtain instructions regarding desired flow rate, viscosity of fluid stored in reservoir, or the like. It will be further understood that information obtained from flow sensor(s) **30** as described herein may also be used to provide diagnostic or other information, such as whether an occlusion exists along the flow path or how much liquid has been dispensed from the reservoir.

Referring now to **FIGs. 7A-B**, schematic hybrids of block diagrams and cross-sectional views of an example of an actuator assembly and pressure regulator are shown. In the depicted embodiment, the pressure regulator **210** includes a housing **209** defining a major chamber. A diaphragm **206** sealingly divides the major chamber into first **204** and second **208** minor chambers. An inlet **201** and an outlet **203** are in fluid communication with the first minor chamber **204**. An inlet **205** and an outlet **207** are in fluid communication with the second minor chamber **208**. As shown in **FIGs. 3-5**, a flow restrictor **220** may be disposed in the fluid flow path between the outlet **203** of the first minor chamber **204** and the inlet **205** of the second minor chamber **208**. Diaphragm **206** is depicted in **FIGs. 5A-B** as a flexible element. However, it will be understood that the diaphragm may be supported by a bellows structure (not shown) to extend in a direction into the first chamber **204** or into the second chamber **208**. In **FIG. 5B**, diaphragm **206** distends further into second chamber **208** relative to first chamber **204** than in **FIG. 5B**. Accordingly the pressure differential between the first chamber **204** and second chamber **208** is different. Specifically, the further diaphragm **206** distends into second chamber **208** the pressure fluid pressure in the second chamber **208** increases relative to the first chamber **204**, thus affecting the pressure differential across the flow restrictor (see e.g. **FIGS. 3-5**) and affecting the flow rate of liquid through the flow path. If diaphragm **206** distends far enough into second chamber **208** diaphragm may engage sealing element **211**, such as an o-ring, to cut off flow through outlet **207**.

The diaphragm **206** may be formed of any suitable material that is impervious to the fluid delivered by the device. In an embodiment, the diaphragm **206** is formed of a thin foil metal such as titanium.

Referring now to the actuator assembly depicted in **FIGs. 7A-B**, assembly includes a motor **424** and an actuator element **426**, such a spring, operably coupled to the motor **424**. The actuator element **426** is also operably coupled to the diaphragm **206** and is capable of causing the diaphragm **206** to move, e.g. flex or expand, in a direction into the first chamber **204** or into the second chamber **208**. The motor **424** may be a stepper motor, a shape memory alloy motor, or the like having a mechanism capable of translating rotational movement of the motor into linear compression or extension of the actuator element **426**, such as a spring. As the actuator element **426** extends or contracts, the position of the diaphragm **206** changes to alter the relative pressure between the first chamber **204** and the second chamber **208**. Such a control mechanism can provide for highly controlled and accurate dispensing of liquid from the infusion device. Such control mechanisms can be employed to ensure the accuracy of constant rate delivery of liquid from the device, if desired, or can be employed to adjust the rate of delivery as desired.

With regard to constant rate delivery, many of the propulsion mechanisms **15** described above with reference to **FIGs. 4** and **5** are intended to supply a constant fluid flow rate but suffer from some drawbacks. For example, fluid rate delivery of liquid propellant based propulsion mechanisms can vary as ambient pressure varies. A patient may experience different flow rates from such devices depending on whether the patient is on an airplane, at 5000 feet (1.5 km) above seal level, at sea level or below sea level. The use of the pressure adjustment actuator mechanism described herein allows the infusion device to compensate for changes in ambient conditions, such as ambient pressure, to ensure a more constant rate of delivery. By way of further example, spring force propulsion mechanisms tend to vary in the force applied around a force exhibited at a certain compression/extension state of the spring. That is, the force applied varies depending on the degree of extension or compression of the spring. In addition, the spring force applied by a spring may vary over time, generally becoming less with time. As such, the delivery rate of liquid from devices employing spring force propulsion mechanisms can vary. Propulsion mechanisms relying on resilient or elastic forces tend to have similar drawbacks as those employing spring force propulsion mechanisms. Regardless of the propulsion mechanism employed, the pressure adjustment actuator mechanisms described herein can be employed to improve consistency and accuracy of liquid dispensing from a constant rate delivery device.

It is worth noting that pressure regulators that do not employ a pressure adjustment actuator mechanism suffer from similar drawbacks to propulsion mechanisms relying on spring forces or resilient or elastic forces. As such, the devices described herein can result in implantable infusion devices with enhanced accuracy and controllability relative to prior devices or concepts.

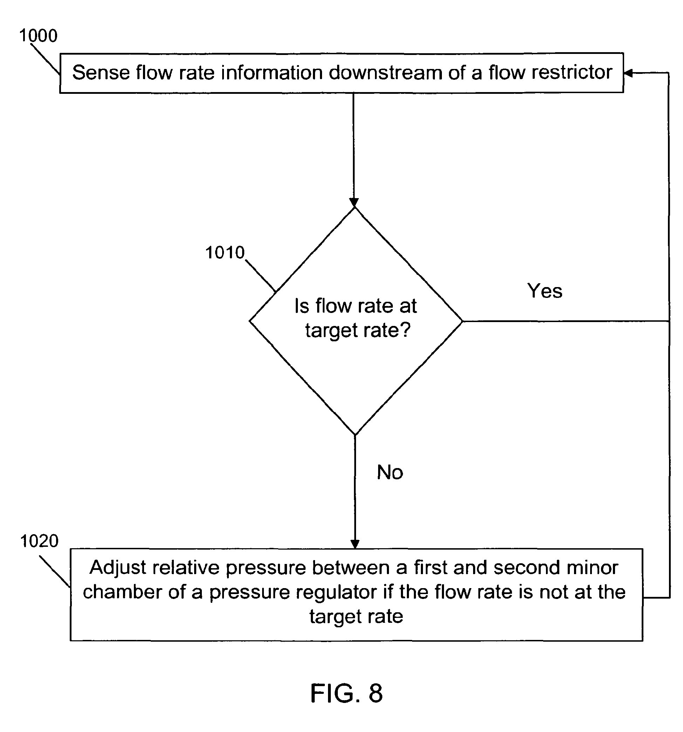

It will be understood that the components described in **FIGs. 2-6** are but examples of components that an implantable infusion device may have and that many other device or system configurations may be employed to carry out the methods described below with regard to **FIGs. 8-9**. However, for the sake of convenience, the discussion that follows with regard to the methods illustrated in the flow diagram of **FIGs. 8-9** will refer to components as described with regard to **FIGs. 2-6**.

Referring now to **FIG. 8** an overview of a method for controlling flow rate is shown. In the depicted method, information regarding flow rate is sensed downstream of a flow restrictor **220** (**1000**). A determination is then made, based at least in part on the sensed information, as to whether the flow rate is at a target rate (**1010**). For example, processor **410** may compare information received from flow sensor **30** to information stored in memory regarding desired parameters to determine whether the flow rate is at the target rate. The method further includes adjusting the relative pressure between a first **204** and second **208** minor chamber of a flow regulator **210** if the flow rate is not at the target rate (**1020**). For example, processor **410** may instruct motor **424** to extend or contract actuator element **426** to adjust the degree to which diaphragm **206** is flexed to adjust the relative pressure between the minor chambers (**204**, **208**). Information regarding the flow rate may continue to be monitored and further adjustments made as appropriate. The rate at which sampling and adjustment occur can be managed to conserve energy consumption.

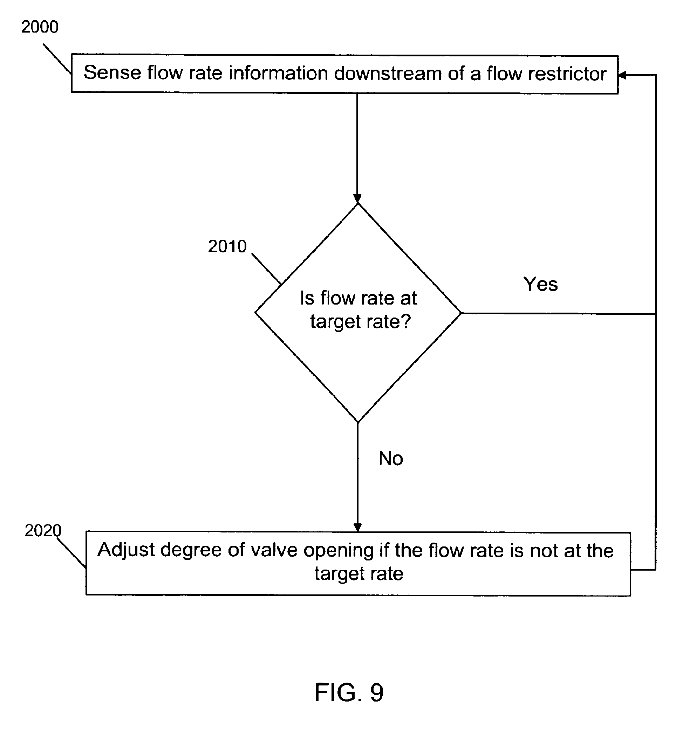

Referring now to **FIG. 9** an overview of a method for controlling flow rate employing a variable valve **700** is shown. In the depicted method, information regarding flow rate is sensed downstream of a flow restrictor **220** (**2000**). A determination is then made, based at least in part on the sensed information, as to whether the flow rate is at a target rate (**2010**). For example, processor **410** may compare information received from flow sensor **30** to information stored in memory regarding desired parameters to determine whether the flow rate is at the target rate. The method further includes adjusting the degree to which a variable valve **700** is open if the flow rate is not at the target rate (**2020**). For example, processor **410** may instruct a motor to extend or contract an actuator element to adjust the degree to which the variable valve **700** is open. Information regarding the flow rate may continue to be monitored and further adjustments made as appropriate.

Thus, embodiments of FLOW SENSOR CONTROLED INFUSION DEVICE are disclosed. One skilled in the art will appreciate that the present invention can be practiced with embodiments other than those disclosed. The disclosed embodiments are presented for purposes of illustration and not limitation, and the present invention is limited only by the claims that follow.

## Claims

1. An implantable infusion device (500) comprising:
an outlet (60) through which a fluid is deliverable; and
a reservoir (10) for containing the fluid; **characterised by** a flow path in fluid communication with the reservoir and the outlet, the flow path including:
(i) a pressure regulator (210) having a housing defining a major chamber and a diaphragm (206) disposed in the housing such that the diaphragm sealingly divides the major chamber into first (206) and second (208) minor chambers; and
(ii) a flow restrictor (220) in fluid communication with the first and second minor chambers of the pressure regulator, wherein the flow restrictor is disposed downstream of the first minor chamber and upstream of the second minor chamber;
a flow sensor (30) configured to detect information regarding flow rate of the fluid downstream of the flow restrictor;
an actuator assembly (420) configured to vary pressure in the first minor chamber relative to pressure in the second minor chamber; and
a processor (410) operably coupled to the flow sensor and the pressure adjustment actuator assembly, wherein the processor is configured to provide instructions to the actuator assembly for adjusting the pressure in the first minor chamber relative to pressure in the second minor chamber based on the information from the sensor to regulate flow rate of the fluid.

2. The device of claim 1, wherein the actuator assembly comprises an actuator element (426) operably coupled to the diaphragm of the pressure regulator and is capable of causing the diaphragm to move to adjust the pressure in the first minor chamber relative to the second minor chamber.

3. The device of claim 2, wherein the actuator assembly further comprises a motor (424) operably coupled to the actuator element and the processor, wherein the motor is capable of receiving the instructions from the processor for adjusting the pressure in the first minor chamber relative to pressure in the second minor chamber of the pressure regulator, and wherein the motor is capable of moving the actuator element to cause the diaphragm to move.

4. The device of claim 3, wherein the motor is capable of translating rotational movement of the motor or a component thereof into linear movement of the actuator element.

5. The device of claim 4, wherein the motor is a stepper motor, or a shape memory motor.

6. The device of claim 1, wherein a sealing element (211) is disposed within the second minor chamber of the pressure regulator, wherein the diaphragm, or a portion thereof, is capable of moving towards and engaging the sealing element to prevent fluid flow out of the second minor chamber.

7. The device of claim 1, wherein the reservoir or a portion thereof comprises a resilient material biased towards an empty position, wherein the reservoir or portion thereof distends when filled and forces fluid to exit the reservoir via the flow path.

8. The device of claim 1, wherein the reservoir is operably coupled to a propulsion mechanism (15) to force fluid to exit the reservoir via the flow path.

9. The device of claim 8, wherein the propulsion mechanism comprises a propellant chamber containing a liquid having a vapor pressure at normal body temperature such that pressure is exerted on the reservoir to force fluid to exit the reservoir via the flow path.

10. An implantable infusion device comprising:
an outlet (60) through which a fluid is deliverable; and
a reservoir (10) for containing the fluid; **characterised by** a flow path in fluid communication with the reservoir and the outlet, the flow path including:
(i) a variable valve (700) configured to allow variable amounts of fluid to pass through the valve; and
(ii) a flow restrictor (220) in fluid communication with and disposed downstream of the variable valve;
a flow sensor (30) configured to detect information regarding flow rate of the fluid downstream of the flow restrictor;
an actuator assembly (420) configured to vary the degree to which the variable valve is open; and
a processor (410) operably coupled to the flow sensor and the actuator assembly, wherein the processor is configured to provide instructions to the actuator assembly for adjusting the degree to which the variable valve is open based on the information from the sensor to regulate flow rate of the fluid.

11. The device of claim 1 or 10, wherein the flow sensor comprises a mass flow sensor (34).

12. A method for controlling flow rate of a fluid through a flow path of an implantable infusion device, the flow path including a flow restrictor and a flow regulator, the flow restrictor including a major chamber sealingly divided into first and second minor chambers by a diaphragm, wherein the flow restrictor is disposed in the flow path between the first and second minor chambers, the method comprising:
sensing flow rate information downstream of the flow restrictor;
determining whether the flow rate is at a target rate based on the sensed information; and
adjusting the relative pressure between the first and second minor chambers of the pressure regulator if the flow rate is not at the target rate.

13. The method of claim 12, wherein adjusting the relative pressure between the first and second minor chambers of the pressure regulator comprises moving the position of the diaphragm.

14. The method of claim 13, wherein moving the position of the diaphragm comprises moving an actuator element operably coupled to the diaphragm.

15. The method of claim 14, wherein moving the actuator element comprises translating rotational movement of a motor, or a component thereof, into linear movement of the actuator element.

## Patentansprüche

1. Implantierbare Infusionsvorrichtung (500), die enthält:
einen Auslass (60), durch den ein Fluid ausgegeben werden kann; und
ein Reservoir (10), um das Fluid zu enthalten; **gekennzeichnet durch**
einen Fließweg in Fluidkommunikation mit dem Reservoir und dem Auslass, wobei der Fließweg enthält:
(i) einen Druckregulierer (210) mit einem Gehäuse, das eine Hauptkammer definiert, und einer Membran (206), die so in dem Gehäuse angeordnet ist, dass die Membran die Hauptkammer in eine erste (206) und eine zweite (208) Unterkammer abdichtend unterteilt; und
(ii) einen Durchflussbegrenzer (220) in Fluidkommunikation mit der ersten und der zweiten Unterkammer des Druckregulierers, wobei der Durchflussbegrenzer stromabseitig der ersten Unterkammer und stromaufseitig der zweiten Unterkammer angeordnet ist;
einen Durchflusssensor (30), der konfiguriert ist, Informationen bezüglich der Durchflussrate des Fluids des Durchflussbegrenzers zu detektieren;
eine Aktuatoranordnung (420), die konfiguriert ist, den Druck in der ersten Unterkammer relativ zu dem Druck in der zweiten Unterkammer zu variieren; und
einen Prozessor (410), der betriebstechnisch an den Durchflusssensor und die Druckeinstellaktuatoranordnung gekoppelt ist, wobei der Prozessor konfiguriert ist, Anweisungen an die Aktuatoranordnung zum Einstellen des Drucks in der ersten Unterkammer relativ zu dem Druck in der zweiten Unterkammer aufgrund der Informationen von dem Sensor einzustellen, um die Durchflussrate des Fluids zu regulieren.

2. Vorrichtung nach Anspruch 1, wobei die Aktuatoranordnung ein Aktuatorelement (426) enthält, das betriebstechnisch an die Membran des Druckregulierers gekoppelt ist und bewirken kann, dass sich die Membran bewegt, um den Druck in der ersten Unterkammer relativ zu der zweiten Unterkammer einzustellen.

3. Vorrichtung nach Anspruch 2, wobei die Aktuatoranordnung ferner einen Motor (424) enthält, der betriebstechnisch an das Aktuatorelement und den Prozessor gekoppelt ist, wobei der Motor Anweisungen von dem Prozessor empfangen kann, um den Druck in der ersten Unterkammer relativ zu dem Druck in der zweiten Unterkammer des Druckregulierers einzustellen, und wobei der Motor das Aktuatorelement bewegen kann, um zu bewirken, dass sich die Membran bewegt.

4. Vorrichtung nach Anspruch 3, wobei der Motor eine Drehbewegung des Motors oder einer seiner Komponenten in eine lineare Bewegung des Aktuatorelements übertragen kann.

5. Vorrichtung nach Anspruch 4, wobei der Motor ein Schrittmotor oder ein Formgedächtnismotor ist.

6. Vorrichtung nach Anspruch 1, wobei das Verschlusselement (211) innerhalb der zweiten Unterkammer des Druckregulierers angeordnet ist, wobei die Membran oder ein Anteil davon sich in seine Richtung bewegen und mit dem Verschlusselement in Eingriff gelangen kann, um zu verhindern, dass das Fluid aus der zweiten Unterkammer herausfließt.

7. Vorrichtung nach Anspruch 1, wobei das Reservoir oder ein Teil davon ein elastisches Material enthält, das in Richtung einer leeren Position voreingestellt ist, wobei sich das Reservoir oder ein Teil davon ausdehnt, wenn es gefüllt ist, und das Fluid zwingt, das Reservoir über den Fließweg zu verlassen.

8. Vorrichtung nach Anspruch 1, wobei das Reservoir betriebstechnisch an einen Antriebsmechanismus (15) gekoppelt ist, um das Fluid zu zwingen, das Reservoir über den Fließweg zu verlassen.

9. Vorrichtung nach Anspruch 8, wobei der Antriebsmechanismus eine Treibmittelkammer enthält, die eine Flüssigkeit enthält, die bei einer normalen Körpertemperatur einen Dampfdruck besitzt, derart, dass Druck auf das Reservoir ausgeübt wird, um das Fluid zu zwingen, das Reservoir über den Fließweg zu verlassen.

10. Implantierbare Infusionsvorrichtung, die enthält:
einen Auslass (60), durch den ein Fluid ausgegeben werden kann; und
ein Reservoir (10) zum Enthalten des Fluids, **gekennzeichnet durch**
einen Fließweg in Fluidkommunikation mit dem Reservoir und dem Auslass, wobei der Fließweg enthält:
(i) ein variables Ventil (700), das konfiguriert ist, variablen Mengen des Fluids zu ermöglichen, **durch** das Ventil zu fließen; und
(ii) einen Durchflussregulierer (220) in Fluidkommunikation mit dem variablen Ventil und stromabseitig von ihm angeordnet;
einen Durchflusssensor (30), der konfiguriert ist, Informationen bezüglich der Durchflussrate des Fluids stromabseitig des Durchflussbegrenzers zu detektieren;
eine Aktuatoranordnung (420), die konfiguriert ist, das Ausmaß, mit dem das variable Ventil geöffnet ist, zu variieren; und
einen Prozessor (410), der betriebstechnisch an den Durchflusssensor und die Akutatoranordnung gekoppelt ist, wobei der Prozessor konfiguriert ist, Anweisungen an die Aktuatoranordnung zum Einstellen des Ausmaßes, mit dem das variable Ventil geöffnet ist, aufgrund der Informationen von dem Sensor zu liefern, um die Durchflussrate des Fluids zu regulieren.

11. Vorrichtung nach Anspruch 1 oder 10, wobei der Durchflusssensor einen Massendurchflusssensor (34) enthält.

12. Verfahren zum Steuern einer Durchflussrate eines Fluids durch einen Fließweg einer implantierbaren Infusionsvorrichtung, wobei der Fließweg einen Durchflussbegrenzer und einen Durchflussregulierer enthält, wobei der Durchflussbegrenzer eine Hauptkammer enthält, die durch eine Membran in eine erste und eine zweite Unterkammer abdichtend aufgeteilt ist, wobei der Durchflussbegrenzer in dem Fließweg zwischen der ersten und der zweiten Unterkammer angeordnet ist, wobei das Verfahren umfasst:
Erfassen von Durchflussrateninformationen stromabseitig des Durchflussbegrenzers;
Bestimmen, ob die Durchflussrate bei einer Sollrate liegt, aufgrund der erfassten Informationen; und
Einstellen des relativen Drucks zwischen der ersten und der zweiten Unterkammer des Druckregulierers, wenn die Durchflussrate nicht bei einer Sollrate liegt.

13. Verfahren nach Anspruch 12, wobei das Einstellen des relativen Drucks zwischen der ersten und der zweiten Unterkammer des Druckregulierers umfasst, die Position der Membran zu bewegen.

14. Verfahren nach Anspruch 13, wobei das Bewegen der Position der Membran umfasst, ein Aktuatorelement, das betriebstechnisch an die Membran gekoppelt ist, zu bewegen.

15. Verfahren nach Anspruch 14, wobei das Bewegen des Aktuatorelements umfasst, die Drehbewegung eines Motors oder einer seiner Komponenten in eine lineare Bewegung des Aktuatorelements zu überführen.

## Revendications

1. Dispositif de perfusion implantable (500) comportant :
un orifice de sortie (60) à travers lequel un fluide peut être délivré ; et
un réservoir (10) pour contenir le fluide ; **caractérisé par** un trajet d'écoulement en communication fluidique avec le réservoir et l'orifice de sortie, le trajet d'écoulement comprenant :
(i) un régulateur de pression (210) ayant un boîtier définissant une chambre principale et un diaphragme (206) disposé dans le boîtier de sorte que le diaphragme divise hermétiquement la chambre principale en une première (206) et une seconde (208) chambres secondaires ; et
(ii) un réducteur de débit (220) en communication fluidique avec les première et seconde chambre secondaires du régulateur de pression, le réducteur de débit étant disposé en aval de la première chambre secondaire et en amont de la seconde chambre secondaire ;
un capteur de débit (30) configuré pour détecter des informations concernant le débit du fluide en aval du réducteur de débit ;
un ensemble d'actionneur (420) configuré pour faire varier la pression dans la première chambre secondaire par rapport à la pression dans la seconde chambre secondaire ; et
un processeur (410) couplé de manière opérationnelle au capteur de débit et à l'ensemble d'actionneur de réglage de pression, dans lequel le processeur est configuré pour fournir des instructions à l'ensemble d'actionneur afin de régler la pression dans la première chambre secondaire par rapport à la pression dans la seconde chambre secondaire sur la base des informations en provenance du capteur pour réguler le débit du fluide.

2. Dispositif selon la revendication 1, dans lequel l'ensemble d'actionneur comporte un élément d'actionneur (426) couplé de manière opérationnelle au diaphragme du régulateur de pression et est capable d'amener le diaphragme à se déplacer pour régler la pression dans la première chambre secondaire par rapport à la seconde chambre secondaire.

3. Dispositif selon la revendication 2, dans lequel l'ensemble d'actionneur comporte en outre un moteur (424) couplé de manière opérationnelle à l'élément d'actionneur et au processeur, dans lequel le moteur est capable de recevoir des instructions en provenance du processeur pour régler la pression dans la première chambre secondaire par rapport à la pression dans la seconde chambre secondaire du régulateur de pression, et dans lequel le moteur est capable de déplacer l'élément d'actionneur pour amener le diaphragme à se déplacer.

4. Dispositif selon la revendication 3, dans lequel le moteur est capable de traduire un mouvement de rotation du moteur ou d'un composant de celui-ci en un mouvement linéaire de l'élément d'actionneur.

5. Dispositif selon la revendication 4, dans lequel le moteur est un moteur pas-à-pas, ou un moteur à mémoire de forme.

6. Dispositif selon la revendication 1, dans lequel un élément d'étanchéité (200) est disposé à l'intérieur de la seconde chambre secondaire du régulateur de pression, dans lequel le diaphragme, ou une partie de celui-ci, est capable de se déplacer vers l'élément d'étanchéité et de mettre en prise celui-ci pour empêcher un écoulement de fluide à l'extérieur de la seconde chambre secondaire.

7. Dispositif selon la revendication 1, dans lequel le réservoir ou une partie de celui-ci comporte un matériau élastique sollicité en direction d'une position vide, dans lequel le réservoir ou une partie de celui-ci se dilate lorsqu'il est rempli et force le fluide à quitter le réservoir via le trajet d'écoulement.

8. Dispositif selon la revendication 1, dans lequel le réservoir est couplé de manière opérationnelle à un mécanisme de propulsion (15) pour forcer le fluide à quitter le réservoir via le trajet d'écoulement.

9. Dispositif selon la revendication 8, dans lequel le mécanisme de propulsion comporte une chambre de propulsion contenant un liquide ayant une pression de vapeur à une température corporelle normale de sorte que la pression est exercée sur le réservoir pour forcer le fluide à quitter le réservoir via le trajet d'écoulement.

10. Dispositif de perfusion implantable comportant :
un orifice de sortie (60) à travers lequel un fluide peut être délivré ; et
un réservoir (10) pour contenir le fluide, **caractérisé par** un trajet d'écoulement en communication fluidique avec le réservoir et l'orifice de sortie, le trajet d'écoulement comprenant :
(i) une soupape variable (700) configurée pour permettre à des quantités variables de fluide de passer à travers la soupape ; et
(ii) un réducteur de débit (220) en communication fluidique avec le la soupape variable et disposé en aval de celle-ci ;
un capteur de débit (30) configuré pour détecter des informations concernant un débit du fluide en aval du réducteur d'écoulement ;
un ensemble d'actionneur (420) configuré pour faire varier le degré auquel la soupape variable est ouverte ; et
un processeur (410) couplé de manière opérationnelle au capteur de débit et à l'ensemble d'actionneur, dans lequel le processeur est configuré pour fournir des instructions à l'ensemble d'actionneur afin de régler le degré auquel la soupape variable est ouverte sur la base des informations en provenance du capteur pour réguler le débit du fluide.

11. Dispositif selon la revendication 1 ou 10, dans lequel le capteur de débit comporte un capteur de débit massique (34).

12. Procédé pour commander le débit d'un fluide à travers un trajet d'écoulement d'un dispositif de perfusion implantable, le trajet d'écoulement comprenant un réducteur de débit et un régulateur de débit, le réducteur de débit comprenant une chambre principale divisée hermétiquement en une première et une seconde chambres secondaires par un diaphragme, dans lequel le réducteur de débit est disposé dans le trajet d'écoulement entre les première et seconde chambres secondaires, le procédé comportant les étapes consistant à :
détecter des informations de débit en aval du réducteur de débit ;
déterminer si le débit est à une vitesse cible sur la base des informations détectées ; et
régler la pression relative entre les première et seconde chambres secondaires du régulateur de pression si le débit n'est pas à la vitesse cible.

13. Procédé selon la revendication 12, dans lequel le réglage de la pression relative entre les première et seconde chambres secondaires du régulateur de pression comporte de déplacer la position du diaphragme.

14. Procédé selon la revendication 13, dans lequel le déplacement de la position du diaphragme comporte de déplacer un élément d'actionneur couplé de manière opérationnelle au diaphragme.

15. Procédé selon la revendication 14, dans lequel le déplacement de l'élément d'actionneur comporte de traduire un mouvement de rotation d'un moteur, ou d'un composant de celui-ci, en mouvement linéaire de l'élément d'actionneur.
